# EUROPÄISCHE PATENTANMELDUNG

(11) **EP 0 657 158 A1**
(43) Veröffentlichungstag der Anmeldung: **14.06.1995**
(21) Anmeldenummer: 94118381.6
(22) Anmeldetag: 23.11.1994
(51) Int. Cl.: A61K 7/13

(54) **Haarfärbemittel**

(30) Priorität: 09.12.1993 DE 4342009
(71) Anmelder: GOLDWELL GmbH, D-64297 Darmstadt (DE)
(72) Erfinder: Lorenz, Heribert, D-64401 Gross-Bieberau (DE); Tennigkeit, Jürgen, Dr., D-64342 Seeheim-Jugenheim (DE)

(57) **Zusammenfassung**

Die Lichtechtheit und Intensität von Haarfärbungen werden wesentlich verbessert, wenn Haarfärbemitteln, die mindestens ein Haarfarbstoffvorprodukt aus der Gruppe 1,4-Diaminobenzol, 1-Methyl-2,5-diaminobenzol, einem Tetraaminopyrimidin und/oder einem Triaminohydroxypyrimidin enthalten, 1,4-Diamino-2-chlorbenzol in Kombination mit minestens einer weiteren Kupplersubstanz, ausgewählt aus der Gruppe α-Naphthol, 1-Methoxy-2-amino-4-(β-hydroxyethylamino)benzol, 2,6-Diaminopyridin, 3-Amino-2-methylamino-6-methoxypyridin, 1-Hydroxy-3-amino-2,6-dichlorbenzol, 1,5-Dihydroxynaphthalin, 1,7-Dihydroxynaphthalin, 1-Ethoxy-2,4-diaminobenzol und/oder 1,3-Bis-(2,4-diaminophenoxy)propan zugesetzt wird.

## Beschreibung

Die vorliegende Erfindung betrifft Haarfärbemittel auf Basis an sich bekannter Entwickler- und Kupplersubstanzen, die Färbungen mit verbesserter Farbbeständigkeit, insbesondere ausgezeichneter Lichtechtheit, ergeben.

Oxidations-Haarfärbemittel bestehen in der Regel aus einer Kombination von Oxidationsfarbstoff-Vorprodukten, d.h. Entwicklersubstanzen und Kupplern, die je nach Art der gewünschten Haarfarbe variieren. Zur Feineinstellung der einzelnen Farben können diese Zusammensetzungen noch Nuanceure, wie direktziehende Farbstoffe, etc. enthalten.

Die weitaus am meisten eingesetzten Entwicklersubstanzen sind 1,4-Diaminobenzol und 1-Methyl-2,5-diaminobenzol. In gewissem Umfang finden auch noch Tetraaminopyrimidine, insbesondere 2,4,5,6-Tetraaminopyrimidin, als Entwicklersubstanzen Verwendung.

Gemäß einer aus der EP-A 467 026 bekanntgewordenen Entwicklung können auch bei Verwendung von Triaminohydroxypyrimidinen, insbesondere 2,4,5-Triamino-6-hydroxypyrimidin, als Entwicklersubstanzen ausgezeichnete Haarfärbungen sowohl in saurem als auch alkalischem Medium erzielt werden.

Eine in Oxidations-Haarfärbemitteln vielfach verwendete Kupplersubstanz ist das 1,3-Diaminobenzol, das als Blaukomponente, insbesondere zur Erzielung von dunklen Farbtönen, besonders im Asch-Bereich, eingesetzt wird.

Es hat sich jedoch gezeigt, daß bei Färbungen, die durch Verwendung von 1,3-Diaminobenzol als Blaukomponente enthaltenden Oxidations-Haarfärbemitteln erzielt wurden, die Lichtechtheit nicht gewährleistet ist und schnell ein Umschlag in eine rötliche Farbtönung auftritt.

Es wurde nun gefunden, daß man diese Nachteile überwinden kann und Haarfarben mit ausgezeichneter Lichtechtheit erhält, wenn man ein Haarfärbemittel verwendet, das neben den genannten Entwicklersubstanzen noch 1,4-Diamino-2-chlorbenzol und mindestens eine weitere Kupplersubstanz aus der Gruppe α-Naphthol, 1-Methoxy-2-amino-4-(β-hydroxyethylamino)benzol, 2,6-Diaminopyridin, 3-Amino-2-methylamino-6-methoxypyridin, 1-Hydroxy-3-amino-2,6-dichlorbenzol, 1,5-Dihydroxynaphthalin, 1,7-Dihydroxynaphthalin, 1-Ethoxy-2,4-diaminobenzol und/oder 1,3-Bis-(2,4-diaminophenoxy)propan enthält. Das 1,4-Diamino-2-chlorbenzol, das auch unter der Bezeichnung o-Chloro-p-phenylendiamin bekannt ist, ist zwar vereinzelt schon als Bestandteil von Haarfärbemitteln vorgeschlagen worden, hat jedoch in der Praxis keine nennenswerte Verwendung gefunden; seine Wirksamkeit in der erfindungsgemäßen Kombination ist daher überraschend.

Der Mengenanteil der Entwicklerkomponenten, die entweder als freie Base oder in Form ihrer Salze, beispielsweise als Hydrochlorid, Sulfat, etc. eingesetzt werden können, liegt zwischen etwa 0,1 und etwa 5 Gewichtsprozent, vorzugsweise etwa 0,5 bis 3 Gew.-%; sie werden gemeinsam mit den Kupplern in einem molaren Verhältnis von etwa 3 : 1 bis etwa 1 : 2, bezogen auf die freie Base, eingesetzt. Das molare Verhältnis von 1,4-Diamino-2-chlorbenzol zu den weiteren Kupplern liegt vorzugsweise im Bereich von 1 : 1 bis 1 : 10.

Erwünschtenfalls können die erfindungsgemäßen Haarfärbemittel noch weitere an sich bekannte Kupplersubstanzen und Nuanceure wie direktziehende synthetische und natürliche Farbstoffe enthalten, soweit dies im Einzelfall zur Erzielung spezieller Farbtöne erwünscht ist.

Als solche seien beispielsweise Resorcin, 2-Methylresorcin, 2- und 4-Chlorresorcin, 3-Aminophenol, 2-Aminophenol, 5-Amino-2-methylphenol, 3-Amino-4-chlor-6-methylphenol, 3-Amino-2-methylphenol, 3,5-Diaminophenylalkanole, insbesondere 3,5-Diaminobenzylalkohol, 2,7-Dihydroxynaphthalin, etc. genannt.

Geeignete direktziehende Farbstoffe sind insbesondere "Arianor"-Farbstoffe sowie verschiedene Nitroverbindungen, beispielsweise 2-Amino-4-nitrophenol und 2-Amino-6-chlor-4-nitrophenol.

Diese Stoffe werden, wenn vorhanden, in niedrigen Konzentrationen, beispielsweise 0,1 bis 1 Gewichtsprozent des Gesamtfärbemittels, eingesetzt.

Die Gesamtmenge des Farbstoffgemisches im Endprodukt beträgt etwa 0,2 bis etwa 6,0 Gewichtsprozent, vorzugsweise etwa 0,5 bis etwa 4 Gew.-%, des Haarfärbemittels.

Zur Herstellung des erfindungsgemäßen Haarfärbemittels werden die Oxidationsfarbstoff-Vorprodukte und ggf. anwesenden direktziehenden Farbstoffe in einen geeigneten kosmetischen Träger eingearbeitet. Solche sind z.B. Emulsionen oder Gele.

Solche Zusammensetzungen und die in ihnen enthaltenen Stoffe sind Stand der Technik und in einschlägigen Monographien, beispielsweise bei K.Schrader, "Grundlagen und Rezepturen der Kosmetika", 2. Auflage (1989, Hüthig Buchverlag), S. 796 bis 815, beschrieben. Auf diese und andere Monographien wird hier ausdrücklich Bezug genommen.

Im folgenden wird die Erfindung anhand von Ausführungsbeispielen illustriert.

### Beispiel 1

In eine Grundlage aus

| | |
|---|---|
| Cetylstearylalkohol | 15,0 (Gew.-%) |
| 1,2-Propylenglycol | 1,0 |
| Ölsäure | 3,0 |
| Cocosmonoethanolamid | 3,0 |
| Stearinsäuremonoethanolamid | 3,0 |
| Natriumlaurylsulfat | 0,5 |
| Natriumsulfit | 1,0 |
| Ammoniumchlorid | 0,5 |
| Ammoniak (25%ig) | 10,0 |
| Parfum | 0,2 |
| Komplexbildner, Stabilisator, Parfum | q.s. |
| Wasser | @ 100,0 |

wurden folgende Oxidationsfarbstoff-Vorprodukte (in Gewichts-%, bezogen auf die Gesamtzusammensetzung) eingebracht:

| Zusammensetzung | 1A | 1B | 2A | 2B |
|---|---|---|---|---|
| 2,5-Diamino-1-methylbenzolsulfat | 0,50 | 0,50 | 0,50 | 0,50 |
| 1-Methoxy-2-amino-4-(β-hydroxyethylamino) benzolsulfat | 0,20 | 0,20 | | |
| 1-Hydroxy-3-amino-2,6-dichlorbenzol | | | 0,20 | 0,20 |
| 1,4-Diamino-2-chlorbenzolsulfat | | 0,20 | | 0,20 |

| Zusammensetzung | 3A | 3B | 4A | 4B |
|---|---|---|---|---|
| 1,4-Diaminobenzol | 0,25 | 0,25 | 0,25 | 0,25 |
| 1-Methoxy-2-amino-4-(β-hydroxyethylamino)benzolsulfat | 0,20 | 0,20 | | |
| 2,6-Diaminopyridin | | | 0,20 | 0,20 |
| 1,4-Diamino-2-chlorbenzolsulfat | | 0,20 | | 0,20 |

| Zusammensetzung | 5A | 5B | 6A | 6B |
|---|---|---|---|---|
| 2,5-Diamino-1-methylbenzolsulfat | 0,12 | 0,12 | 0,30 | 0,30 |
| Resorcin | 0,07 | 0,07 | | |
| 3-Aminophenol | 0,05 | 0,05 | | |
| 1-Methoxy-2-amino-4-(β-hydroxyethylamino)benzolsulfat | 0,05 | 0,05 | | |
| 1,4-Diamino-2-chlorbenzolsulfat | | 0,08 | | 0,15 |
| 1,5-Dihydroxynaphthalin | | | 0,15 | 0,15 |

Bei den Beispielen 1B, 2B, 3B, 4B, 5B und 6B handelt es sich um erfindungsgemäße Zusammensetzungen; bei den Beispielen 1A, 2A, 3A, 4A, 5A und 6A um solche nach dem Stand der Technik.

Die Produkte wurden jeweils mit gleichen Anteilen 6%iger Wasserstoffperoxid-Lösung vermischt und auf Haarsträhnen aus menschlichem Haar, Büffelhaar und auf Woll-Läppchen aufgebracht. Nach 30-minütigem Einwirken wurden die Strähnen gewaschen, getrocknet und 8 Stunden UV-bestrahlt. Die danach vorgenommene visuelle Wertung zeigte bei den mit den erfindungsgemäßen Zusammensetzungen behandelten Strähnen eine glänzende Farbe, während die mit 1A, 2A, 3A, 4A, 5A und 6A behandelten Strähnen ausgebleicht wirkten und keinen Glanz aufwiesen.

Die Farbtöne der einzelnen Zusammensetzungen waren:
- Nr. 1:: Mittleres Cendré-Blau
- Nr. 2:: Blaustichiger Aschton
- Nr. 3:: Mittleres Cendré-Blau
- Nr. 4:: Kräftiger Aschton
- Nr. 5:: Helles Aschblond
- Nr. 6:: Cendré-Blond

### Beispiel 2

In eine Grundlage aus

| | |
|---|---|
| Cetylstearylalkohol | 10,00 (Gew.-%) |
| Stearinmonoethanolamid | 2,00 |
| Cocosmonoethanolamid | 2,00 |
| Polyethylenglycol-5-cocosamid | 6,00 |
| Ölsäure | 1,20 |
| Natriumsulfit | 0,30 |
| Natriumlaurylsulfat | 0,50 |
| Ammoniumchlorid | 0,30 |
| Mangandioxid | 0,12 |
| Kaliumjodid | 0,01 |
| Komplexbildner, Stabilisator, Parfum | q.s. |
| Wasser | @ 100,0 |

wurden folgende Oxidationsfarbstoff-Vorprodukte (in Gew.-%, bezogen auf die Gesamtzusammensetzung) eingebracht:

| Zusammensetzung | 7A | 7B | 8A | 8B |
|---|---|---|---|---|
| 2,5,6-Triamino-4-hydroxypyrimidsulfat | 0,50 | 0,50 | 0,50 | 0,50 |
| α-Naphthol | 0,15 | 0,15 | | |
| 1,4-Diamino-2-chlorbenzolsulfat | | 0,15 | | 0,25 |
| 1-Methoxy-2-amino-4-(β-hydroxyethylamino)benzolsulfat | | | 0,25 | 0,25 |

Die Färbungen und Auswertungen derselben erfolgten, wie in Beispiel 1 beschrieben, wobei das Verhältnis von Farbstoffmischung zu einer 2%igen wäßrigen H₂O₂-Lösung bei 1 : 2 lag. Dabei war die Lichtechtheit der erzielten Blondfärbung (Nr. 7) bzw. hellen Blaufärbung (Nr. 8) mit den erfindungsgemäßen Zusammensetzungen 7B und 8B gegenüber den mit 7A und 7B behandelten Haarsträhnen und Woll-Läppchen deutlich besser.

## Patentansprüche

1. Haarfärbemittel, enthaltend eine Kombination aus
a) mindestens einem Oxidations-Farbstoffvorprodukt der Gruppe 1,4-Diaminobenzol, 1-Methyl-2,5-diaminobenzol, einem Tetraaminopyrimidin und/oder einem Triaminohydroxypyrimidin und deren wasserlöslichen Salzen,
b) 1,4-Diamino-2-chlorbenzol bzw. dessen wasserlöslichen Salzen; und
c) mindestens einer Kupplersubstanz, ausgewählt aus der Gruppe α-Naphthol, 1-Methoxy-2-amino-4-(β-hydroxyethylamino)benzol, 2,6-Diaminopyridin, 3-Amino-2-methylamino-6-methoxypyridin, 1-Hydroxy-3-amino-2,6-dichlorbenzol, 1,5-Dihydroxynaphthalin, 1,7-Dihydroxynaphthalin, 1-Ethoxy-2,4-diaminobenzol und/oder 1,3-Bis-(2,4-diaminophenoxy)propan.

2. Haarfärbemittel nach Anspruch 1, dadurch gekennzeichnet, daß es als Triaminohydroxypyrimidin das 2,5,6-Triamino-4-hydroxypyrimidin enthält.
